# EUROPÄISCHE PATENTSCHRIFT

(11) **EP 3 661 465 B1**
(45) Veröffentlichungstag und Bekanntmachung des Hinweises auf die Patenterteilung: **08.09.2021**
(21) Anmeldenummer: 18759033.6
(22) Anmeldetag: 25.07.2018
(51) Int. Cl.: A61F 2/90, D04C 1/06, A61F 2/848, A61F 2/04, A61B 17/11

(54) **STENT**
STENT
STENT

(30) Priorität: 01.08.2017 DE 102017117439
(43) Veröffentlichungstag der Anmeldung: 10.06.2020
(73) Patentinhaber: Novatech SA, 13705 La Ciotat Cedex (FR)
(72) Erfinder: SIEGNER, Georg, 12159 Berlin (DE)
(74) Vertreter: Griepenstroh, Jörg
(86) Internationale Anmeldenummer: PCT/EP2018/070218
(87) Internationale Veröffentlichungsnummer: WO 2019/025265

(56) Entgegenhaltungen:
- EP-A1- 1 849 440
- EP-A1- 2 918 251
- US-A1- 2017 014 133

## Beschreibung

Die Erfindung betrifft einen Stent mit den Merkmalen im Oberbegriff des Patentanspruchs 1.

Ein endoskopisch implantierbarer Stent zur Platzierung in einer Körperhöhle kann beispielsweise eine Verbindung zwischen dem Magen und einer Pseudozyste herstellen. Hierzu wird ein kleines Loch in der Magenwand und in der Wand der Pseudozyste geschnitten. Durch dieses Loch wird das Einführsystem für den Stent geschoben und der Stent in dem Loch platziert. Der Stent hat zwei aufgespreizte Seiten (Flanken) und einen zylindrischen Mittelteil. Er ähnelt in seiner Kontur daher einem Diabolo. Die Flanken haben die Funktion, den Stent daran zu hindern, aus dem Implantationsort herauszurutschen. Der zylindrische Teil hält den Durchgang zwischen der Pseudozyste und dem Magen offen, damit Material aus der Pseudozyste abfließen kann. Der zylindrische Teil ist in dem Loch der Magenwand und der Pseudozyste platziert, während die Flanken zum einen im Magen und zum anderen in der Pseudozyste platziert sind. Da das präparierte Loch zum Einführen des Stents möglichst klein sein soll, wird der Durchmesser des zylindrischen Teils des Stents direkt nach der Implantation bedingt durch den kleinen Durchmesser des Lochs sehr klein zusammengedrückt. Dies führt bei der herkömmlichen Webung eines Drahtgerüst des Stents dazu, dass auch die Flanken des Stents zusammengedrückt werden, d. h. im Durchmesser reduziert werden. Diese Durchmesserreduzierung kann so weit gehen, dass ein Herausrutschen des Stents aus dem Implantationsort möglich wird. Die gerade Webung der Stents führt leider dazu, dass sich ein Stent, wenn er an einer Stelle zusammengedrückt wird, sich an angrenzenden Stellen ebenfalls im Durchmesser verringert. Das ist zwar hilfreich beim manuellen Crimpen, kann jedoch bei den vorstehend beschriebenen Stents am Implantationsort zu Problemen führen. US 2017/014133 A1 beschreibt einen Stent nach dem Oberbegriff des Anspruchs 1. Hiervon ausgehend liegt der Erfindung die Aufgabe zugrunde, einen Stent mit einem im Durchmesser kleineren Mittelabschnitt und mit im Durchmesser vergrößerten Endabschnitten aufzuzeigen, welcher ein Zusammendrücken des mittleren Teils ermöglicht, ohne dass dabei der Durchmesser der Endabschnitte in gleichem Maße reduziert wird.

Diese Aufgabe ist bei einem Stent mit den Merkmalen des Patentanspruchs 1 gelöst. Die Unteransprüche betreffen zweckmäßige Weiterbildungen der Erfindung.

Der erfindungsgemäße Stent besitzt einen mittleren Längenabschnitt, der vorzugsweise zylindrisch ist. An den mittleren Längenabschnitt schließen sich zwei Endabschnitte an. Der Stent besitzt ein tragendes Gerüst aus einem verformbaren Drahtgewebe. Der Stent ist insbesondere selbstexpandierend. Er kann von einem Cover aus einem Kunststoff umgeben sein, welches zusammen mit dem tragenden Gerüst expandiert wird. Das Cover hält das Drahtgewebe zudem in Form, in dem Sinne, dass benachbarte Drähte über das flexible Cover miteinander verbunden sind. Das Cover folgt der Bewegung des tragenden Drahtgerüstes.

Der erfindungsgemäße Stent kann nach der Implantierung im Durchmesser expandieren. Dementsprechend besitzt er anfangs einen kleinen Durchmesser. Der Stent besitzt im expandierten Zustand einen Durchmesser, der im Bereich der Endabschnitte größer ist als im mittleren Längenabschnitt. Der Stent ist daher gewissermaßen Hantel- oder Diabolo-förmig. Die Endabschnitte können auch als Flanken bezeichnet werden, die dafür vorgesehen sind, den Stent in einer Wand zu halten, wobei der Stent eine Öffnung in der Wand durch setzt und wobei jeweils eine Flanke in einem Lumen angeordnet ist, dass an die Wand grenzt.

Erfindungsgemäß ist vorgesehen, dass der gewebte Draht Zickzack-förmig von einem äußeren Ende des einen Endabschnitts kommend hin in Richtung des äußeren Endes des anderen Endabschnitts und von dort zurück zum ursprünglichen Endabschnitt verläuft. Auch der Rückweg ist Zickzack-förmig. Die Wendepunkte der Zickzack-Form sind jeweils im Übergang von einem Endabschnitt in den mittleren Längenabschnitt angeordnet, so dass sich die Umfangsrichtung, in welcher der gewebte Draht positioniert ist, sich jeweils im Übergang von einem Endabschnitt in den mittleren Längenabschnitt ändert. Dadurch ergibt sich, dass sich der Draht auf dem Rückweg zum ersten Endabschnitt in dem mittleren Längenabschnitt kreuzt. Dadurch ergibt sich eine 8-förmige Schlaufe mit 2 geschlossenen Augen, allerdings mit eckiger Kontur. Im Unterschied zu einer geraden Webung, bei welcher die Umfangsrichtung des gewebten Drahtes gleich bleibt, wird durch die spezielle Zickzack-Webtechnik erreicht, dass die Bewegung beim Zusammendrücken des zylindrischen mittleren Längenabschnitts über das Drahtgeflecht in die Endabschnitte umgelenkt wird, dass dort eine Bewegung in Gegenrichtung ausgelöst wird. Das bewirkt im Bereich der Endabschnitte eine radiale Aufweitung der äußeren Enden, d.h. eine Spreizung. Die Spreizung bewirkt wiederum eine sichere Arretierung des Stents in dem Durchgang, so dass der Stent wesentlich sicherer gehalten ist und nicht aus dem Durchgang herausrutschen kann.

Die erfindungsgemäße Webung koppelt die Bewegung der Endabschnitte von dem mittleren Längenabschnitt ab, in dem Sinne, dass die Bewegung bei einer Durchmesserreduzierung in der Mitte nicht gleichsinnig sondern gegensinnig ist. Die trichterförmigen Endabschnitte des Stents falten sich direkt bei der Implantation zu ihrer normalen Größe auf. Die spezielle Webung erreicht dabei, dass sich die Endabschnitte des Stents beim Zusammendrücken des Mittelteils sogar verstärkt öffnen, was mit einer geraden Webung nicht möglich ist.

Es wird als vorteilhaft angesehen, wenn der Zickzack-förmige Verlauf des Drahtes auf dem Rückweg vom zweiten Endabschnitt zum ersten Endabschnitt spiegelbildlich zum vorhergehenden Zickzack-förmigen Verlauf des Drahtes auf dem Hinweg vom ersten Endabschnitt zum zweiten Endabschnitt ist. Die spiegelsymmetrische Ausbildung bewirkt, dass sich die angrenzenden Bereiche der Endabschnitte identisch verformen. Die gegenläufige Bewegung des Stents in den Endabschnitten ist besonders gleichmäßig, wenn die Zickzack-förmige Webung spiegelbildlich angeordnet ist.

Die Erfindung geht davon aus, dass die Webung gewissermaßen endlos ist und sich von einem Anfangspunkt an einem äußeren Ende des ersten Endabschnittes beginnend bis zu einem Endpunkt erstreckt, der mit dem Anfangspunkt identisch sein kann. Das Webmuster mit den 8-förmigen Laschen wird um einen bestimmten Umfangsbereich versetzt wiederholt. Sofern von sich schneidenden Zickzack-förmigen Verlaufen gesprochen wird, ist damit der Verlauf des Drahtes von einem zum anderen Endabschnitt und wieder zurück gemeint. Im Anschluss wiederholt sich dasselbe Muster. Durch Überlappungen und Kreuzungspunkte wird ein hinreichend dichtes und tragfähiges Drahtgewebe geschaffen. Das Muster kann sich beispielsweise alle 30° wiederholen. Dementsprechend sind die besagten Anfangspunkte und Umlenkpunkte von Schlaufe zu Schlaufe um 30° zueinander versetzt. Engere oder weitere Einteilungen sind selbstverständlich möglich.

In vorteilhafter Weiterbildung der Erfindung ist der eingeschlossene Winkel zwischen zwei Schenkeln des Drahtes beim Übergang von einem Endabschnitt in den mittleren Längenabschnitt in einer Abwicklung des Stents kleiner als 90°. Es handelt sich um einen spitzen Winkel. Für den spitzen Winkel ist es notwendig, dass der Draht im ersten Endabschnitt bereits in einem, spitzen Winkel zur Längsachse auf den ersten Umlenkpunkt zuläuft.

Bezogen auf die Umfangsrichtung ist der Rücksprung, welchen der Draht nach dem ersten Umlenkpunkt im mittleren Längenabschnitt macht, größer als der Umfangsbereich, den der Draht im ersten Endabschnitt zurücklegt. Der Draht ist in seinem dritten Schenkel des Zickzack-förmigen Verlaufs dementsprechend wieder in Umfangsrichtung orientiert. Am äußeren Ende des zweiten Endabschnitts wird der Draht umgelenkt. Wegen der gewünschten Achssymmetrie ist der Verlauf des Drahtes auf dem Rückweg genau umgekehrt zum Hinweg. Der letzte Schenkel auf dem Rückweg ist demzufolge ebenso spiegelsymmetrisch zum ersten Schenkel des Hinweges. Damit ein umfangseitiger Versatz der Schlaufen entsteht, kreuzen sich der erste Schenkel des Hinweges und der letzte Schenkel des Rückweges im ersten Längenabschnitt. Der Endpunkt der ersten Schlaufe ist daher in Umfangsrichtung versetzt. Beginnend von diesem Startpunkt kann nun die nächste Schlaufe gelegt werden, beispielsweise um 30° in Umfangsrichtung versetzt.

Im Unterschied zu einer im Wesentlichen geraden oder umlaufenden Wicklung erstreckt sich eine einzelne Schlaufe über einen relativ engen Umfangsbereich, der zumindest kleiner als 360° ist. Der Draht erstreckt sich im mittleren Längenabschnitt vorzugsweise über einen Umfangsbereich von mehr als 90°, insbesondere über einen Umfangsbereich von 115°. Hierdurch wird im mittleren Längenabschnitt eine hinreichende Abdeckung und bevorzugt eine große Überdeckung mit benachbarten Schlaufen erreicht, so dass die nach radial außen gerichteten Offenhaltungskräfte des Stents durch entsprechend enge Webung hinreichend groß sind. Je größer der Umfangsbereich ist, über den sich der Draht im mittleren Längenabschnitt erstreckt, desto dichter ist die Webung.

Vorzugsweise sind die Endabschnitte und damit die sich aufstellenden Flanken in der Abwicklung länger als der mittlere Längenabschnitt. Das bedeutet, dass sich die Flanken im expandierten Zustand weiter aufstellen können als der mittlere Längenabschnitt, wenn man davon ausgeht, dass sich alle Längenabschnitte gleichmäßig dehnen. Ein kürzerer mittlerer Längenabschnitt bedeutet, dass der Draht im mittleren Längenabschnitt eine größere Steigung in Bezug auf die Umfangsrichtung besitzt als in den beiden Endabschnitten. Das ist die bevorzugte Ausführungsform. Trotz der bevorzugt längeren Endabschnitte und des kürzeren Mittelabschnitts soll der Draht im mittleren Längenabschnitt länger sein als in jeweils einem der benachbarten Endabschnitte. Auch dieses Merkmal trägt dazu bei, dass es viele Kreuzungsstellen im mittleren Längenabschnitt gibt, so dass die Webdichte hier besonders groß ist. Der Stent erhält dadurch die Eigenschaft im Unterschied zu weniger dichten Webungen schwerer zusammengedrückt werden zu können. Seine Aufhaltekraft ist größer. Wie bereits erwähnt, wird es als besonders bevorzugt angesehen, wenn benachbarte bzw. aufeinander folgende Schlaufen bzw. Zickzack-förmige Wicklungen in Umfangsrichtung um 30° zueinander versetzt sind. Wenn gleichzeitig der mittlere Längenabschnitt sich über 115° erstreckt, führt das dazu, dass jeder mittlere Schenkel des Zickzack-förmig gewobenen Drahtes von sechs weiteren mittleren Schenkeln geschnitten wird, die in entgegengesetzte Richtung verlaufen. Da dies für jeden der Schenkel im mittleren Längenabschnitt gilt, ergibt sich dort ein sehr dichtes Webmuster.

Die Anzahl der Schnittpunkte im ersten und zweiten Längenabschnitt ist geringer, weil der Draht dort eine geringere Steigung hat und sich daher auch über einen kleineren Umfangsbereich erstreckt. Die Webung ist offener, flexibler, nicht so dicht, was allerdings für die Funktion einer Flanke eines Diabolo-förmigen Stents völlig ausreichend und sogar wünschenswert ist.

Die Erfindung wird nachfolgend anhand eines in den Zeichnungen schematisch dargestellten Ausführungsbeispieles näher erläutert. Es zeigen:
- Figur 1: das Webmuster eines Pseudozysten-Stents in der Abwicklung (Stand der Technik),
- Figur 2: die Abwicklung eines erfindungsgemäßen Webmusters eines Pseudozysten-Stents;
- Figur 3: einen gewobenen Stent in einer Seitenansicht (Stand der Technik);
- Figur 4: den Stent der Figur 3, in der Mitte zusammengedrückt;
- Figur 5: zwei Stents (links Stand der Technik, rechts erfindungsgemäß);
- Figur 6: die Stents der Figur 5, teilweise im zusammengedrückten Zustand;
- Figur 7: die Freisetzung zweier Stents (links Stand der Technik, rechts erfindungsgemäße Webung), wobei der 1. Endabschnitt freigesetzt wird;
- Figur 8: die Stents der Figur 7, nachdem die Hälfte der Stens freigesetzt ist;
- Figur 9: die Stents der Figur 7, nachdem dreiviertel ihrer Länge freigesetzt ist und
- Figur 10: die Stents der Figur 7 nach dem vollständigen Freisetzen.

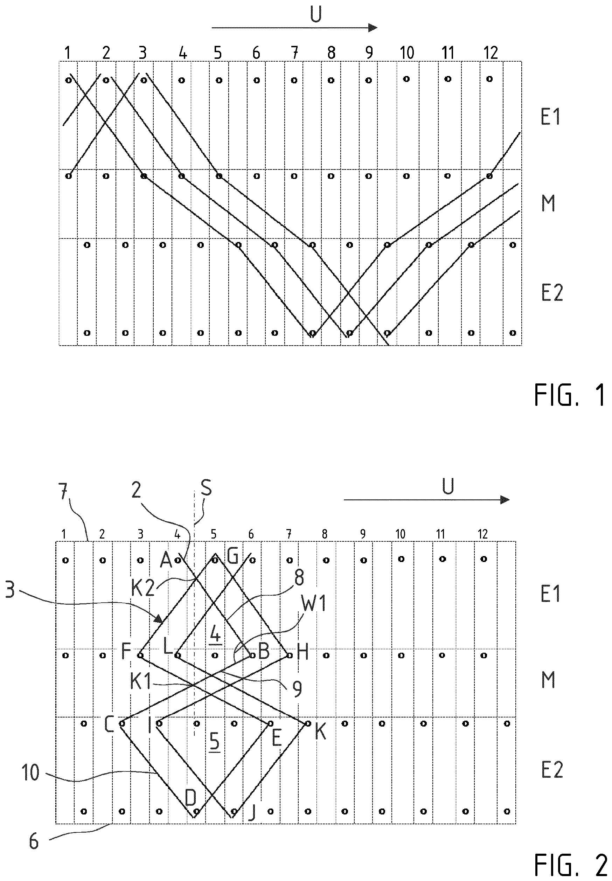

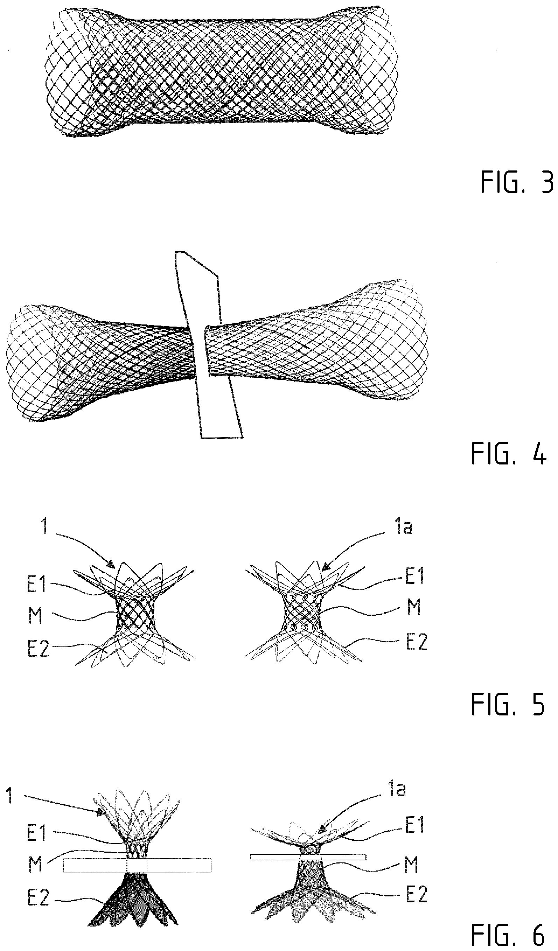

Figur 1 zeigt das Webmuster eines Pseudozysten-Stents 1 als Stand der Technik. Die Darstellung ist eine Abwicklung des Stents 1. Der zugehörige Stent 1 ist in den Figuren 5 bis 10 jeweils links dargestellt. Der erfindungsgemäße Stent 1A ist in der Bildebene der Figuren 5 bis 10 jeweils rechts dargestellt. Das zugehörige Webmuster zum erfindungsgemäßen Stent Zar ist in Figur 2 dargestellt.

### Zunächst zum Stand der Technik:

Figur 3 zeigt einen gewobenen Stent zum Stand der Technik. Die Form des Stents ist im Wesentlichen zylindrisch. Er besitzt einen mittleren Längenabschnitt und zwei kürzere Endabschnitte, wobei die Endabschnitte im Durchmesser erweitert sind. Dieser Stent ist prinzipiell nach dem Webmuster gemäß Figur 1 gewebt. Figur 4 zeigt den Stent der Figur 3, wie er in der Mitte zusammengedrückt ist. Dies führt zu einer Durchmesserreduzierung in unmittelbarer Nähe des mittleren Bereichs, aber auch über die nahezu gesamte Länge des Stents. Zwar ist zu erkennen, dass die Durchmesserreduzierung zu den Enden hin abnimmt, allerdingst ist im Wesentlichen der gesamte mittlere Längenabschnitt im Durchmesser reduziert, obwohl es nur eine punktuelle Belastung in der Mitte gibt.

Das Webmuster, das diesen Effekt hervorruft, ist in der Abwicklung in Figur 1 gezeigt. Die Abwicklung zeigt drei Längenabschnitte des Stents. Es gibt jeweils einen Endabschnitt E1, E2 und zwischen den Endabschnitten E1, E2 einen etwas kürzeren mittleren Längenabschnitt M. Der mittlere Längenabschnitt M ist etwa nur halb so lang wie ein Endabschnitt E1, E2. Der Draht verläuft konsequent in Umfangsrichtung U, d. h. in Richtung des eingezeichneten Pfeils. Die Zahlen 1 bis 12 bezeichnen einzelne Abschnitte des Umfanges U. Zwischen zwei Abschnitten erstreckt sich ein Winkelbereich von 30°. Es ist zu erkennen, dass die Drahtwebung über den Umfang betrachtet nie die Richtung ändert. Auch wenn die Steigung des Drahtes im mittleren Längenabschnitt M sich ändert in dem Sinne, dass sie etwas größer ist als in dem benachbarten Endabschnitten E1, E2, so wird der Draht immer in einer Richtung weiter über den Umfang U geführt. Der Draht wird immer von links nach rechts in der Bildebene geführt. An den Kreuzungsstellen des Drahtes werden die Drähte einmal über- und einmal untereinander hergeführt, so dass eine Webung entsteht.

Dieser Stent 1, wie er in Figur 5 links gezeigt ist, hat die Eigenschaft, dass sich die gewissermaßen als Flanken ausgebildeten Endabschnitte E1, E2 beim Einsetzen in eine im Durchmesser kleine Wandöffnung, d. h. beim Komprimieren des mittleren Längenabschnitts M abflachen. Das zeigt Figur 6. In Figur 5 ist derselbe Stent 1 im entspannten Zustand dargestellt. Die Flanken bzw. Endabschnitte E1, E2 haben einen steileren (größeren) Anstellwinkel zur Längsachse des Stents. Bei Komprimierung des Mittelabschnitts ist der Anstellwinkel wesentlich kleiner, so dass der Stent 1 aus der Öffnung leichter herausrutschen kann.

Der erfindungsgemäße Stent 1a gemäß Figur 5 (rechte Abbildung) sieht im entspannten Zustand bezüglich seiner Konturgebung genauso aus wie der linke Stent 1 zum Stand der Technik. Für im Wesentlichen gleiche Komponenten des erfindungsgemäßen Stents 1a werden nachfolgend die zu dem bereits erläuterten Stent 1 genannten Bezugszeichen verwendet. Insbesondere hat der mittlere Längenabschnitt M dieselbe Länge. Auch die Flankenwinkel der Endabschnitte E1, E2 sind identisch.

Figur 6 zeigt, dass der erfindungsgemäße Stent 1a, der ebenfalls in seinem mittleren Längenabschnitt M komprimiert wird, in der Einbausituation ein ganz anderes Verhalten aufweist als der in der Bildebene linke Stent. Die Flankenwinkel sind nicht kleiner geworden, sondern im Vergleich zu der entspannten Ausgangsposition gemäß Figur 5 sogar größer. Besonders deutlich wird das an dem in der Bildebene der Figur 6 oberen Endabschnitt E1. Er ist nahezu um 90° nach außen aufgestellt, so dass der Stent 1a keinesfalls durch die symbolisch dargestellte Öffnung hindurchrutschen kann. Die in Figur 6 dargestellte Einschnürung liegt in diesem Ausführungsbeispiel näher an dem oberen Endabschnitt E1 als an dem unteren Endabschnitt E2. Daher ist der Endabschnitt E1 auch weiter aufgefächert als der andere Endabschnitt E2. Das bedeutet, dass sich die Einschnürung umso stärker auf das Auffächern der Endabschnitte auswirkt, je näher die Einschnürung zu einem der Endabschnitte E1, E2 benachbart ist. Der Stent ist bezüglich seiner Mittelquerebene spiegelsymmetrisch ausgebildet, so dass sich derselbe Effekt auch ergeben würde, wenn der Stent 1a umgekehrt in die Öffnung bzw. Engstelle eingesetzt worden wäre, bzw. wenn der Stent mit seinem 2. Endabschnitt E2 näher zur Öffnung rutscht. Es ist festzustellen, dass der erfindungsgemäße Stent 1a daher in der Einbaulage bessere Eigenschaften aufweist, wenn er bereichsweise oder über die gesamte Länge des mittleren Längenabschnitts eingeschnürt wird. Auch in diesem Fall würden sich die Einschnürungen positiv auf die Anstellwinkel der Endabschnitte E1, E2 auswirken, indem die Endabschnitte steiler aufgestellt werden. Seine Eigenschaften sind ohne Einschnürung genauso gut wie bei dem Stent 1 gemäß dem Stand der Technik.

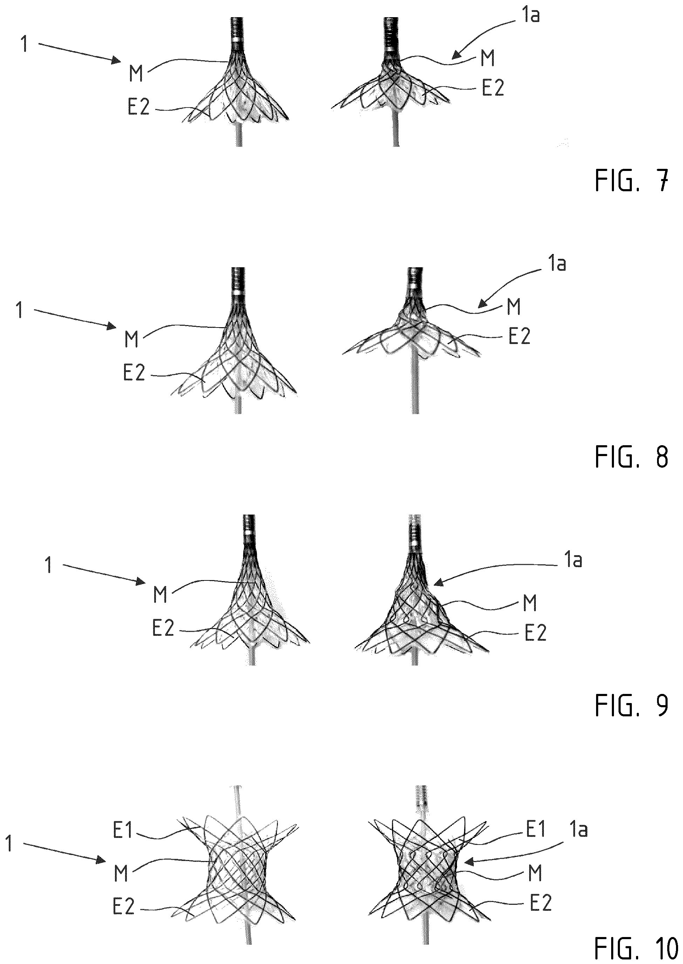

Die Figuren 7 bis 10 zeigen, wie der Stent 1 gemäß dem Stand der Technik und der erfindungsgemäße Stent 1a freigesetzt werden. In Figur 7 ist nur der jeweils zuerst austretende Endabschnitt E2 freigesetzt. Hier ist bereits zu erkennen, dass die noch bestehende Einschnürung im mittleren Längenabschnitt M sich derart auf den erfindungsgemäßen Endabschnitt E2 auswirkt, dass dieser einen steileren Anstellwinkel zur Längsachse hat und daher über eine kürzere Länge weiter aufspreizt als der vergleichbare Endabschnitt E2 gemäß dem Stent 1 zum Stand der Technik.

Figur 8 zeigt die beiden Stents 1, 1a, die jeweils zur Hälfte freigesetzt worden sind. Auch hier ist noch einmal deutlich zu erkennen, dass der in der Bildebene rechte, erfindungsgemäße Stent 1a bezüglich seines Endabschnittes E2 deutlich steiler aufgefächert ist als bei einem Stent 1 gemäß dem Stand der Technik.

Figur 9 zeigt die beiden Stents 1, 1a, nachdem sie zu dreiviertel freigesetzt worden sind. Da der unmittelbar benachbarte Bereich des Endabschnitts E2, also der mittlere Längenabschnitt M, der nur durch den noch nicht freigesetzten Endabschnitt E1 gehalten ist und daher weniger stark komprimiert ist, ist auch der Endabschnitt E2 nicht mehr so steil aufgefächert. Deutlich zu erkennen ist allerdings, dass der mittlere Längenabschnitt M insgesamt betrachtet einen größeren Außendurchmesser hat als der erfindungsgemäße Stent 1. Das bedeutet, dass der noch komprimierte Teil des noch nicht freigegebenen Endabschnitts E1 des Stents 1a sich deutlich weniger auf den Durchmesser des mittleren Längenabschnitts auswirkt als es beim Stand der Technik der Fall ist. Die beiden Endabschnitte E1, E2 und der mittlere Längenabschnitt M sind hinsichtlich ihrer gegenseitigen Beeinflussung ihrer Außenkonturen stärker voneinander entkoppelt als es bei dem Stent 1 zum Stand der Technik der Fall ist.

Nach der kompletten Freigabe der Stents 1,1a, wie es in Figur 10 gezeigt ist, ergibt sich abgesehen von dem anderen Webmuster die im Wesentlichen identische Außenkontur. Da die Längen, Proportionen und Durchmesser der einzelnen Längenabschnitte der Stents 1, 1a identisch sind, ergibt sich im Wesentlichen die gleiche Außenkontur. Im Ergebnis hat der in der Bildebene rechte Stent 1a gemäß der Erfindung jedoch die besseren Eigenschaften in der implantierten Position, da das Herausrutschen des Stents aus dem Implantationsort effektiver verhindert wird als bei einem Stent gemäß dem Stand der Technik.

Einzelheiten zu dem Webmuster werden nachfolgend anhand der Figur 2 erläutert. Der Draht wird Zickzack-förmig über mehrere Umlenkpunkte A-L geführt. Es ist eine im Wesentlichen 8-förmige Schlaufe 3 zu erkennen, die zwei geschlossene Augen 4, 5 hat. Die Figur 2 zeigt zwei dieser 8-förmigen Schlaufen 3 nebeneinander, weil sich das Webmuster jeweils um in Umfangsrichtung U versetzt wiederholt. Das Webmuster kann auch als Zickzack-förmiges Muster bezeichnet werden. Die Zickzack-Form ergibt sich dadurch, dass der Draht 2 vom ersten Endabschnitt E1 kommend schräg in Bezug auf die Umfangsrichtung U vom 1. Umlenkpunkt A (Startpunkt) zum 2. Umlenkpunkt B geführt wird. Am Umlenkpunkt B, der sich an der Grenze zum mittleren Längenabschnitt M befindet, wird die Webrichtung umgekehrt und der Draht 2 verläuft entgegen der Umfangsrichtung U vom 2. Umlenkpunkt B zum 3. Umlenkpunkt C an der Grenze zum 2. Endabschnitt E2. Dieser Rückschritt ist in Umfangsrichtung betrachtet sogar größer als Umfangsabstand vom Umlenkpunkt A zum Umlenkpunkt B. In diesem Ausführungsbeispiel wurden von dem Draht 2 von Umlenkpunt A nach Umlenkpunt B 60° überdeckt, während beim Rückschritt von Umlenkpunt B nach Umlenkpunt C ein Umfangsbereich von 115° überdeckt wurde. Schließlich führt der Draht 2 in Umfangsrichtung vom Umlenkpunkt C zum nächsten Umlenkpunkt D am äußeren Ende 6 des zweiten Endabschnittes E2. Damit ist die eine Hälfte der 8-förmigen Schlaufe 3 beschrieben. Ausgehend vom Umlenkpunkt D wird der Draht 2 gespiegelt wieder zum 1. Endabschnitt E1 zurückgeführt. Weil vom Umlenkpunkt C zum Umlenkpunkt D ein Winkel von 60° überschritten wurde, wird der Draht 2 nunmehr ebenfalls in Umfangsrichtung um 60° versetzt zu dem Umlenkpunkt E im Übergang zum mittleren Längenabschnitt M geführt. Von dort erfolgt wiederum der Rückschritt entgegen der Umfangsrichtung U zum nächsten Umlenkpunkt F im Übergang zum ersten Endabschnitt E1. Von dort führt der Draht 2 wiederum in gespiegelter Anordnung zum äußersten Ende 7 des ersten Endabschnitts E1 (Umlenkpunkt G). Der Umlenkpunt G ist der Endpunkt der 1. Schlaufe 3. Da Startpunkt und Endpunkt nicht identisch sind, beginnt die nächste Schlaufe 3 um 30° versetzt entsprechend dem Umfangsabstand der Umlenkpunkte A und G am äußersten Ende 7 des ersten Endabschnitts E1. Der umgelenkt. Die Liste damit der Startpunkt für die 2. Schlaufe. Dieses Webmuster wiederholt sich entlang der Umlenkpunkte H bis L solange bis der Draht 2 nach 360° wieder beim ersten Umlenkpunkt A ankommt. Dann ist der Stent 1 komplett gewebt.

In nicht näher dargestellter Weise wird der Draht an den Kreuzungspunkten des weiteren Verlaufes über- und untereinander durchgeführt, so dass sich ein Drahtgewebe ergibt. Erfindungsgemäß ist vorgesehen, dass aufgrund der 8-förmigen Schlaufe 3 ein Kreuzungspunkt K1 im mittleren Längenabschnitt M liegt. Ein weiterer Kreuzungspunkt K2 ergibt sich im ersten Endabschnitt E1, weil erster Umlenkpunkt A und letzter Umlenkpunkt G nicht deckungsgleich sind. Die Symmetrieachse S durch die beiden Kreuzungspunkte K1, K2 verläuft parallel zur Längsachse, so dass die Schlaufe 3 gewissermaßen achsparallel zum Stent angeordnet ist.

Figur 2 zeigt ferner, dass das in der Bildebene untere Auge 5 der Schlaufe 3 größer ist als das obere Auge 4. Das ist darauf zurückzuführen, dass die Umlenkpunkte C, E zwischen dem mittleren Längenabschnitt M und dem zweiten Endabschnitt E2 weiter auseinander liegen als im Übergang zum ersten Endabschnitt E1. Dennoch ist die Schlaufe 3 spiegelsymmetrisch bezüglich der Spiegelachse S durch die Schnittpunkte K1 und K2. Die Zickzack-förmige Schlaufe 3 schließt beim Übergang von einem Endabschnitt E1 zu dem mittleren Längenabschnitt M einen Winkel W1 ein, der kleiner als 90° ist. Der Winkel W1 ist hier beispielhaft zwischen dem ersten Schenkel 8 und dem zweiten Schenkel 9 eingezeichnet, wobei sich der erste Schenkel 8 zwischen den Umlenkpunkten A und B und der zweite Schenkel zwischen den Umlenkpunkten B und C erstreckt. Beide Schenkel 8, 9 verlaufen zwischen aufeinanderfolgenden Umlenkpunkten A-L gerade. Die Zickzack-Form des Stents 1a bezieht sich daher auf den Verlauf des Drahtes 2 zwischen den Umlenkpunkten A-L.

Da der Draht 2 im mittleren Längenabschnitt M einen größeren Umfangsbereich überstreicht als in einem Endabschnitt E1, E2, besitzt der Draht 2 im mittleren Längenabschnitt M eine größere Steigung in Bezug auf die Umfangsrichtung U. Der nicht näher dargestellte eingeschlossene Winkel zwischen dem zweiten Schenkel 9 und dem Pfeil, der in Umfangsrichtung U weist, ist daher kleiner als der Winkel zwischen den übrigen Schenkeln des Drahtes 2 in den Endabschnitten E1, E2 und der Umfangsrichtung U. Daher ist der Schenkel 9 im mittleren Längenabschnitt M auch länger als ein Schenkel 8 im ersten Endabschnitt E1 bzw. ein entsprechender Schenkel 10 im zweiten Endabschnitt E2.

Die Figuren 6 bis 10 zeigen zwischen den einzelnen Schlaufen der Stents 1, 1a Grauschattierungen. Diese sollen verdeutlichen, dass der Draht in einen Mantel eingebettet ist, der von dem Drahtgeflecht unterstützt wird und der Bewegung des Drahtes 2 folgt. Der Mantel besteht insbesondere aus Kunststoff. Der Draht besteht aus einem biokompatiblen Werkstoff, insbesondere einer Formgedächtnislegierung wie beispielsweise Nitinol.

### Bezugszeichen:

- 1 -: Stent
- 1a -: Stent
- 2 -: Draht
- 3 -: Schlaufe
- 4 -: Auge
- 5 -: Auge
- 6 -: äußeres Ende von E2
- 7 -: äußeres Ende von E1
- 8 -: Schenkel
- 9 -: Schenkel
- 10 -: Schenkel

- A bis L -: Umlenkpunkte
- E1 -: erster Endabschnitt
- E2 -: zweiter Endabschnitt
- M -: mittlerer Längenabschnitt
- S -: Spiegelachse
- U -: Umfangsrichtung
- W1 -: Winkel

## Patentansprüche

1. Stent mit einem mittleren Längenabschnitt (M) und zwei Endabschnitten (E1, E2)) und mit einer elastisch verformbaren Drahtgewebe, das es dem Stent (1) ermöglicht, von einem kleinen Durchmesser auf einen größeren Durchmesser zu expandieren, wobei der Durchmesser der Endabschnitte (E1, E2) im expandierten Zustand größer als im mittleren Längenabschnitt (M) ist, **dadurch gekennzeichnet, dass** der Draht (2) Zickzack-förmig von einem äußeren Ende (7) des einen Endabschnitts (E1) kommend hin in Richtung des äußeren Endes (6) des anderen Endabschnitts (E2) und von dort Zickzack-förmig zurück zum ursprünglichen Endabschnitt (E1) verläuft, wobei sich jeweils beim Übergang von einem Endabschnitt (E1, E2) in den mittleren Längenabschnitt (M) die Umfangsrichtung des gewebten Drahtes (2) ändert, so dass sich der Draht (2) auf dem Rückweg zum ersten Endabschnitt (E1) in dem mittleren Längenabschnitt (M) kreuzt und insgesamt eine 8-förmige Schlaufe (3) mit zwei geschlossenen Augen (3,4) bildet, wobei mehrere miteinander verbundene Schlaufen (3) über den Umfang (U) zueinander versetzt angeordnet sind.

2. Stent nach Anspruch 1, **dadurch gekennzeichnet, dass** der Zickzack-förmige Verlauf des Drahtes (2) auf dem Rückweg vom zweiten Endabschnitt (E2) zum ersten Endabschnitt (E1) spiegelbildlich zum vorhergehenden Zickzack-förmigen Verlauf des Drahtes (2) auf dem Hinweg vom ersten Endabschnitt (E1) zum zweiten Endabschnitt (E2) ist, wobei eine Spiegelachse (S) parallel zu einer Längsrichtung des Stents (1a) verläuft.

3. Stent nach Anspruch 1 oder 2, **dadurch gekennzeichnet, dass** der eingeschlossene Winkel (W1) zwischen zwei Schenkeln (8,9) des Drahtes (2) beim Übergang von einem Endabschnitt (E1, E2) in den mittleren Längenabschnitt (M) in einer Abwicklung des Stents (1a) weniger als 90° beträgt.

4. Stent nach einem der Ansprüche 1 bis 3, **dadurch gekennzeichnet, dass** der Draht (2) im mittleren Längenabschnitt (M) eine größere Steigung in Bezug auf die Umfangsrichtung (U) besitz, als in den beiden Endabschnitten (E1, E2).

5. Stent nach Anspruch 4, **dadurch gekennzeichnet, dass** sich der Draht (2) im mittleren Längenabschnitt (M) über einen Umfangsbereich (U) von mehr als 90° erstreckt.

6. Stent nach einem der Ansprüche 1 bis 5, **dadurch gekennzeichnet, dass** der Draht (2) im mittleren Längenabschnitt (M) länger ist als in jeweils einem der benachbarten Endabschnitte (E1, E2).

7. Stent nach einem der Ansprüche 1 bis 4, **dadurch gekennzeichnet, dass** einander benachbarte Wicklungen um 30° in Umfangsrichtung (U) zueinander versetzt angeordnet sind.

8. Stent nach einem der Ansprüche 1 bis 7, **dadurch gekennzeichnet, dass** das Drahtgewebe in einen Mantel eingebettet ist.

## Claims

1. Stent having a central length section (M) and two end sections (E1, E2), and having an elastically deformable woven wire mesh which enables the stent (1) to expand from a small diameter to a larger diameter, wherein the diameter of the end sections (E1, E2) is larger than in the central length section (M) in the expanded state, **characterised in that** the wire (2) extends in a zig-zag manner, coming from an outer end (7) of one end section (E1) towards the outer end (6) of the other end section (E2), and from there back to the original end section (E1) in a zig-zag manner, wherein the circumferential direction of the woven wire (2) changes at the transition from an end section (E1, E2) to the central length section (M), such that the wire (2) intersects on the return path to the first end section (E1) in the central length section (M) and, as a whole, forms a figure 8-shaped loop (3) having two closed eyes (3, 4), wherein a plurality of interconnected loops (3) are arranged offset to each other over the circumference (U).

2. Stent according to claim 1, **characterised in that** the zigzag-shaped course of the wire (2) on the return path from the second end section (E2) to the first end section (E1) is a mirror image of the preceding zigzag-shaped course of the wire (2) on the outward path from the first end section (E1) to the second end section (E2), wherein a mirror axis (S) extends parallel to a longitudinal direction of the stent (1a).

3. Stent according to claim 1 or 2, **characterised in that** the enclosed angle (W1) between two branches (8, 9) of the wire (2) at the transition from an end section (E1, E2) to the central length section (M) is less than 90° in a developed view of the stent (1a).

4. Stent according to any of claims 1 to 3, **characterised in that** the wire (2) in the central length section (M) has a pitch which, with respect to the circumferential direction (U), is greater than in the two end sections (E1, E2).

5. Stent according to claim 4, **characterised in that** the wire (2) extends in the central length section (M) over a circumferential range (U) of more than 90°.

6. Stent according to any of claims 1 to 5, **characterised in that** the wire (2) is longer in the central length section (M) than in each of the adjacent end sections (E1, E2).

7. Stent according to any of claims 1 to 4, **characterised in that** mutually adjacent windings are offset relative to each other by 30° in the circumferential direction (U).

8. Stent according to any of claims 1 to 7, **characterised in that** the woven wire mesh is embedded in a sheath.

## Revendications

1. Stent avec une section longitudinale médiane (M) et deux sections d'extrémité (E1, E2) et avec un grillage métallique élastiquement déformable qui permet au stent (1) de s'étendre d'un petit diamètre à un plus grand diamètre, dans lequel le diamètre des sections d'extrémité (E1, E2) dans l'état étendu est plus grand que dans la section longitudinale médiane (M), **caractérisé en ce que** le fil (2) se déroule en formant un zigzag d'une extrémité extérieure (7) d'une section d'extrémité (E1) en allant en direction de l'extrémité extérieure (6) de l'autre section d'extrémité (E2) et, de là, en formant un zigzag pour revenir à la section d'extrémité d'origine (E1), dans lequel la direction circonférentielle du fil tissé (2) change respectivement lors du passage d'une section d'extrémité (E1, E2) à la section longitudinale médiane (M), de sorte que le fil (2) traverse la section longitudinale médiane (M) sur le chemin du retour vers la première section d'extrémité (E1) et forme au total une boucle en forme de 8 (3) avec deux yeux fermés (3, 4), dans lequel plusieurs boucles (3) reliées les unes aux autres sont agencées de manière décalée les unes par rapport aux autres sur la circonférence (U).

2. Stent selon la revendication 1, **caractérisé en ce que** le parcours formant un zigzag du fil (2) sur le chemin du retour de la seconde section d'extrémité (E2) vers la première section d'extrémité (E1) est une image miroir du parcours formant un zigzag précédent du fil (2) sur le chemin de la première section d'extrémité (E1) vers la seconde section d'extrémité (E2), dans lequel un axe miroir (S) se déroule parallèlement à une direction longitudinale du stent (1a).

3. Stent selon la revendication 1 ou 2, **caractérisé en ce que** l'angle compris (W1) entre deux branches (8, 9) du fil (2) au passage d'une section d'extrémité (E1, E2) dans la section longitudinale médiane (M) lors d'un traitement du stent (1a) est inférieur à 90°.

4. Stent selon l'une quelconque des revendications 1 à 3, **caractérisé en ce que** le fil (2) possède une inclinaison plus grande par rapport à la direction circonférentielle (U) dans la section longitudinale médiane (M) que dans les deux sections d'extrémité (E1, E2).

5. Stent selon la revendication 4, **caractérisé en ce que** le fil (2) se déploie sur une zone circonférentielle (U) de plus de 90° dans la section longitudinale médiane (M).

6. Stent selon l'une quelconque des revendications 1 à 5, **caractérisé en ce que** le fil (2) est plus long dans la section longitudinale médiane (M) que dans chacune des sections d'extrémité adjacentes (E1, E2).

7. Stent selon l'une quelconque des revendications 1 à 4, **caractérisé en ce que** des enroulements adjacents sont agencés de manière décalée les uns par rapport aux autres de 30° dans la direction circonférentielle (U).

8. Stent de l'une quelconque des revendications 1à 7, **caractérisé en ce que** le grillage métallique est incorporé dans une gaine.
